(19) Europäisches Patentamt
European Patent Office
Office européen des brevets

(11) **EP 4 685 225 A1**

(12) **EUROPEAN PATENT APPLICATION**

(43) Date of publication:
**28.01.2026   Bulletin 2026/05**

(21) Application number: 24190312.9

(22) Date of filing: **23.07.2024**

(51) International Patent Classification (IPC):
*C12N 1/14* (2026.01)       *A23J 1/00* (2006.01)
*A23J 3/20* (2006.01)       *A23K 10/12* (2016.01)
*A23K 10/30* (2016.01)      *A23L 7/104* (2016.01)
*A23L 31/00* (2016.01)

(52) Cooperative Patent Classification (CPC):
**C12N 1/14; A23J 1/008; A23J 3/20; A23K 10/12;
A23K 10/30; A23L 7/104; A23L 31/00**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **UAB Biohifas
10224 Vilnius (LT)**

(72) Inventors:
- **Martusevice, Paulina
  Kaunas (LT)**
- **Borkertas, Simas
  Kaunas (LT)**
- **Byckovas, Tomas
  Vilnius (LT)**

(74) Representative: **AAA Law
A. Gostauto 40B
03163 Vilnius (LT)**

(54) **FUNGAL BIOMASS PRODUCTION USING FOOD BY-PRODUCTS**

(57)    Described herein are various embodiments of the method for production of a composition comprising a fungal biomass. The method can generally include providing a lignocellulose substrate, inoculating the lignocellulose substrate with at least one mycelium-producing fungi, growing the mycelium-producing fungi to produce a fungal biomass, and harvesting the fungal biomass and the remaining substrate. In some embodiments, the lignocellulose substrate comprises food by-product. In some embodiments, use of the composition comprising the harvested fungal biomass and the remaining substrate in the production of food products is provided.

Fig. 1

**Description**

FIELD OF INVENTION

The present application relates to methods of using food industry by-products as lignocellulose substrates, in particular, for producing fungal biomass using solid-state fermentation method.

BACKGROUND OF INVENTION

Globally, it is estimated that as much as 32% of potential food is lost along the entire food supply chain. Meanwhile, the fruit, vegetable and grain industries generate the largest share of food waste [1]. In a typical case, plant-based food waste includes husks, bran, pomace, etc. Most of this biomass consists of lignocellulose, which is a complex matrix of cellulose, hemicellulose, lignin, arabinoxylans, pectins and secondary metabolites. Polysaccharides connect phenolic compounds, peptides and amino acids using cross-covalent, ionic and other bonds. The amounts of bioactive compounds accumulated in plant extracts are ten or more times higher than in their products. There are some uses of food by-products where the by-products are, for example, mechanically processed and re-introduced directly into the production of new food products. However, the components in lignocellulose in the food by-products are not naturally released in digestive systems, and their extraction requires organic solvents: methanol, ethanol, acetone, $CO_2$ extractions, etc. [2]. For this reason, in order to create higher added value product, commercially available enzymes are incorporated to extractions achieve higher cell-wall permeability.

Therefore, there is a need in the art for further food industry by-product valorization options as well as efficient and/or sustainable ways to extract or use the bioactive components from existing food industry by-products.

SUMMARY OF INVENTION

The present invention disclosure solves the problems as set out above. First of all, the present disclosure provides the possibility of food by-products valorization. In particular, the food by-products are used as a lignocellulosic substrate for growth of mycelium-producing fungi and obtain a composition comprising a fungal biomass. The use of the food by-product for this purpose is environmentally and economically more attractive than providing artificial or pure substrates that are specially produced for growing fungi and producing fungal biomass. In particular, filamentous fungi that produce large amounts of mycelium, and therefore mycoprotein, are grown on a lignocellulose substrate made of food by-product. Growing filamentous fungi as provided herein is further advantageous in view of efficient biodegradation and digestion of the food by-product-based lignocellulose substrate, as the filamentous fungi are capable of breaking down lignocellulose of the substrate and produce nitrogen compounds such as amino acids, and, as a result, proteins. Thus, a combination of food by-product-based lignocellulose substrate and using fungi of filamentous genus allows using considerably lower amounts of synthetic nutrient additives (or none at all in specific cases) when a protein product comprising fungal biomass is produced. In addition to the above, another advantage when filamentous fungi are grown on the food by-product lignocellulose substrate as provided by the present invention disclosure is that there is no need to separate the grown fungal biomass from the lignocellulose substrate. This provides higher added nutritional value to the resulting composition comprising fungal biomass, while at the same time less method steps are required in order to produce and harvest a protein product (i.e. composition comprising fungal biomass), as opposed to other currently used methods of solid-state fermentation.

Accordingly, the present invention provides the following:

In a first aspect, the present invention disclosure provides a method for the production of a composition comprising a fungal biomass, comprising the steps: a) providing a lignocellulose substrate, b) inoculating the lignocellulose substrate with at least one mycelium-producing fungi, c) growing the mycelium-producing fungi to produce a fungal biomass, d) harvesting the fungal biomass and the remaining substrate.

In some embodiments, the lignocellulose substrate used in the method according to the invention, comprises particles having a diameter of 1.5 mm or less.

In some embodiments, the lignocellulose substrate comprises food by-product, preferably, a plant-based food by-product. In some embodiments, the food by-product is selected from plant husks, bran, pomace, oil press cake, hulls, leaves, spent grains, peels and combinations thereof. In some embodiments, the food by-product is selected from brewer's and other crop industries spent grain, hemp press cake, sugar beet press cake, carrot press cake, beet root press cake, coffee hulls and used grounds, legumes and potatoes starch-rich liquids, and combinations thereof. In some embodiments, the lignocellulose substrate is comprised of food by-product, preferably, of a plant-based food by-product.

In some embodiments, in the method according to the present invention, the lignocellulose substrate is further supplemented with additional nutrients, selected from carbohydrate source, nitrogen source, macronutrients, micronutrients, enzymes, other additives and combinations thereof. In some embodiments, the carbohydrate source is selected from glucose and starch and combination thereof. In some embodiments, the nitrogen source is selected from yeast extract, brewers spent grains, legumes by-products and legumes starches and combinations thereof. In some embodiments, the macronutrients are selected from plant origin food by-products such as residues, hulls, seed cakes, leaves, stems, roots, husks, peels, spent grains, pomace from fruit and vegetables, crops such as rice, wheats, maize, oats, buckwheat, sorghum and other, legumes, coffee, cacao, olives, sunflower and other oils food industries.

In some embodiments, the lignocellulose substrate is supplemented with amylolytic and/or cellulolytic enzymes. In some embodiments, the amylolytic and/or cellulolytic enzymes are selected from α-Amylases (EC 3.2.1.1), β-amylase (EC 3.2.1.2), glucoamylase (EC 3.2.1.3), α-glucosidase (EC 3.2.1.20), amylopullulanase (EC 3.2.1.41), cyclodextrin glycosyltransferase (EC 2.4.1.19), (endo-(1,4)-β-d-glucanase (EC 3.2.1.4), exo-(1,4)-β-d-glucanase (EC 3.2.1.91), β-glucosidases (EC 3.3.1.21), β-glucosidases (EC 3.3.1.21), and combination thereof.

In some embodiments, the lignocellulose substrate is supplemented with additives selected from yeast extract, magnesium ions, calcium ions, zinc ions, and combination thereof.

In some embodiments, the method according to the present invention further comprises a step of sterilizing the substrate, wherein the step is performed before step b).

In some embodiments, the method according to the present invention further comprises a step of aerating the substrate, wherein the step is performed before step b).

In some embodiments, the method according to the present invention further comprises a step of placing the substrate on a solid support, wherein the step is performed before or after step b). In some embodiments, the solid support is configured to facilitate air circulation to the substrate.

In some embodiments, the method according to the present invention further comprises a step of stabilizing the harvested fungal biomass and the remaining substrate.

In some embodiments, in the method of the present invention, the lignocellulose substrate is inoculated with one or more mycelium-producing fungi species. In some embodiments, the lignocellulose substrate is inoculated with more than one mycelium-producing fungi species. In some embodiments, the mycelium-producing fungi is selected from *Aspergillus* genus, *Rhizopus* genus, *Fusarium* genus, *Pleurotus* genus, *Lentinus* genus, *Hericium* genus and combinations thereof. In some embodiments, the mycelium-producing fungi is *Fusarium* genus.

In a second aspect, a composition comprising a fungal biomass, obtained by a method according to the present invention is provided. In some embodiments, the composition comprises a fungal biomass of mycelium-producing fungi and lignocellulose substrate, wherein the composition comprises at least 30% (w/w) of the fungal biomass. In some embodiments, said composition comprises at least 12% (w/w) of protein.

In a third aspect, use of the composition comprising a fungal biomass, obtained by a method according to the present invention, in the production of food, nutraceuticals, functional food, pharmaceutical products, protein powder, nutritional supplement, higher-added nutritional value products, products claimed as source of proteins, ready-to go meals, pet food, feed, food and feed ingredients is provided. In some embodiments, a food product comprising the composition comprising a fungal biomass, obtained by a method according to the present invention, is provided.

DESCRIPTION OF DRAWINGS

**Figure 1.** Flow diagram depicting a method according to one of preferred embodiments.

**Figure 2.** Sheet pan preparation for mycelium based biomass production on a substrate.

**Figure 3.** Sterilized and aerated substrate placed on sheet pan.

**Figure 4.** Filamentous fungi ready to harvest phase.

**Figure 5.** Filamentous fungi coverage in the substrate (50-95%).

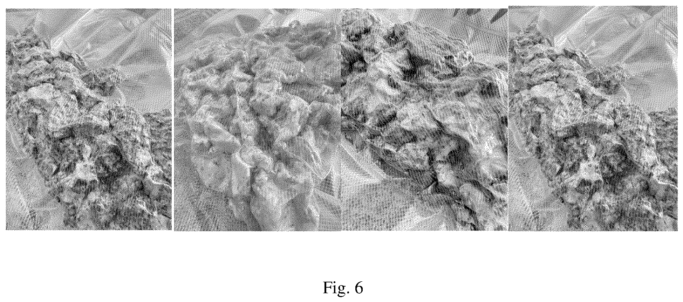

**Figure 6.** Vacuum sealed dried higher protein content biomass.

DETAILED DESCRIPTION OF THE INVENTION

The present invention will be described in the following disclosure. It is to be understood that all the combinations of features are envisaged.

Firstly, the present invention disclosure provides a method for the production of a composition comprising a fungal biomass, comprising the steps: a) providing a lignocellulose substrate, b) inoculating the lignocellulose substrate with at least one mycelium-producing fungi, c) growing the mycelium-producing fungi to produce a fungal biomass, d) harvesting the fungal biomass and the remaining substrate.

As used herein, "lignocellulose substrate" refers to a solid phase substrate containing lignocellulose. A lignocellulose substrate is preferably produced or obtained from lignocellulosic feedstock. An exemplary lignocellulose feedstock that can be used as lignocellulose substrate in the method according to the present invention comprises food by-products. The term "food by-product" as used herein, alternatively also called "food side-stream", "food industry side stream", "food by-product stream" or "food industry by-product", refers to residues, hulls, seed cakes, leaves, stems, roots, inflorescences, husks, peels, spent grains, pomace, liquids from fruit and vegetables, crops (rice, wheats, maize, oats, buckwheat, sorghum and other grains), legumes, coffee, cacao, olives, sunflower and other oils, dairy and feedstock by-products from food industries. In some embodiments, the food by-product is plant-based food by-product, such as brewers spent grain, oat, rice and other crop spent grain, coffee bean hulls or coffee chalks, rapeseed pomace or press cake, linseeds' pomace or press cake, hemp (e.g. *Cannabis sativa*) seed or protein cake, sugar beet press cake, carrot pomace or press cake, beet root press cake and similar.

Preferably, in order to obtain a lignocellulose substrate, the lignocellulose feedstock is processed, for example, the lignocellulose feedstock is homogenized. Methods for homogenizing solid or semi-solid materials are well known in the art, for example, a grain mill can be used. As a result of processing, such as homogenization, the resulting lignocellulose substrate is in the form of particles. The diameter of the particles may be from 5 mm to 1.5 mm, in some examples, the diameter may be from 2 mm to less than 1.5 mm. In preferred embodiments, the lignocellulose substrate used in the method according to the present invention comprises particles having a diameter of 1.5 mm or less. For example, the diameter can be from 1.5 mm to 0.5 mm, e.g. particles may have a diameter of 1.5 mm, 1.4 mm, 1.3 mm, 1.2 mm, 1.1 mm, 1 mm, 0.9 mm, 0.8 mm, 0.7 mm, 0.6 mm or 0.5 mm. In some embodiments, the diameter of particles of lignocellulose substrate is less than 1.5 mm. A lignocellulose substrate having particles with the diameter of 1.5 mm or less, compared to a lignocellulose substrate with larger particles, wherein the two substrates have been otherwise made of the same amount of lignocellulose feedstock, provides larger areas of useful surface for the mycelium-producing fungi to grow on, as more nutrients present in the substrate are accessible to the growing mycelium. Methods of providing particles of selected diameter are well known in the art, for example, a grain mill with a fractionation function of a specific particles size can be used, for example, a grain mill with fractionation size of <1.5 mm.

The method according to the present invention provides a composition comprising a fungal biomass, wherein advantageously, the resulting fungal biomass - mycelium - is harvested together with the lignocellulose substrate portion (i.e. the remaining lignocellulose substrate) that remains unused/undigested by the fungi after incubation and growing of the fungal biomass, that is there is no need to separate the biomass/ mycelium from the remaining substrate. This allows having less steps in preparation of the protein product, as well as allows having less waste that is discarded after the fermentation.

In some embodiments, in a method according to the present invention, the lignocellulose substrate comprises food by-product. Preferably, the food by-product is plant-based food by-product, such as food by-product from food processing industry such as oil industry, brewers industry, spent grain and other crop industries. Examples of food by-products obtained in oil food industry are from olives, seed or nuts, for example seed or nut press cakes, such as rapeseed, linseed, sunflower, hemp seed press cakes. The examples of spent grain include brewer's spent grain, spent grains from oat, rice beverages and other. Food side stream includes residues, hulls, seed cakes, leaves, stems, roots, husks, peels, spent grains, pomace from fruit and vegetables, crops (rice, wheats, maize, oats, buckwheat, sorghum and other), legumes, coffee, cacao, olives, sunflower and other oils food industries.

In some embodiments, lignocellulose substrate made from food by-product comprises about 30-60% (w/w) of hemicellulose, cellulose and lignin, taken together. Preferably, lignocellulose substrate comprises about 40-42% (w/w) hemicellulose, cellulose and lignin, such composition being advantageous as a well accepted and digestable substrate for fungi growth and fermentation of mycoproteins. In some embodiments, lignocellulose substrate comprises about 30-60% (w/w) of hemicellulose, cellulose and lignin, taken together, wherein about 15-25% (w/w) is cellulose and lignin. Preferably, about 20% (w/w) is cellulose and lignin.

In some embodiments, in a method of the present invention, the food by-product is selected from husks, bran, pomace, oil press cake and combinations thereof. Preferably, the food by-product is plant-based and is selected from plant husks, bran, pomace, oil press cake, hulls, leaves, spent grains, peels and combinations thereof.

In some embodiments, in a method according to the present invention, the food by-product is selected from brewers and other crop industries spent grain, hemp press cake, sugar beet press cake, carrot press cake, beet root press cake, coffee hulls and used grounds, legumes and potatoes starch-rich liquids, and combinations thereof. In some embodiments, brewer's spent grain is used. In some embodiments, a combination of brewer's spent grain with hemp press cake, sugar beet press cake, carrot press cake or beet root press cake is used. In some embodiments, the mass ratio of brewer's spent grain and a respective food by-product is 1:3, 1:4 or 1:5, preferably 1:4. In some embodiments, brewers spent grain (moisture 5%) starch content is in range 5-12%, preferably 9%, nitrogen content in range 3.5-7%, preferably 4,5%, phosphorus content in range 0.5-1%, preferably 0,65%, zinc content (BSG 12% moisture) in range 70-200 mg/kg, preferably 15 mg/kg, calcium content in range 1500-3500 mg/kg, preferably 2500 mg/kg, magnesium content in range 2000-3500 mg/kg, preferably 2500 mg/kg. In some embodiments, other food by-products (moisture content in range 5-11%, preferred 8%) for example, hemp (for example, *Cannabis sativa*) press cake, sugar beet press cake starch content is in range 1-80%, preferably 20%, nitrogen content in range 0.5-10%, preferably 4,5%, phosphorus content in range 0.1-3%, preferably 1%, zinc content in range 5-200 mg/kg, preferably 100 mg/kg, calcium content in range 400-8600 mg/kg, preferably 2500 mg/kg, magnesium content in range 300-7000 mg/kg, preferably 2000 mg/kg.

Exemplary methodologies to determine appropriate compositions are provided as below:

Starch content, % - EB/152/2009, appendix III, K part;

Phosphorus content, % - EB/152/2009, appendix III, N part;

Nitrogen, %- EB/152/2009, appendix III, C part;

Zinc (Zn), mg/kg - LST EN ISO 6869:2003;

Calcium (Ca), mg/kg - LST EN ISO 6869:2003;

Magnesium (Mg), mg/kg - LST EN ISO 6869:2003;

Hemicellulose, cellulose, lignin content - Ankom Technology

(https://www.ankom.com/applications/crude-and-detergent-fiber-analysis)

In some embodiments, the lignocellulose substrate is prepared from a food industry by-product by drying the selected food by-product or a mixture thereof about 2-15% of moisture content, preferably to 5-8% of moisture content and homogenizing it to particles, preferably, the particle diameter is about 1.5 mm or less. Thus the food by-product is converted into a substrate (i.e. lignocellulose substrate) suitable for growing mycelium-producing fungi. Said substrate may be further processed before use, for example, it may be sterilized, aerated, supplemented with additional nutrients, or its humidity may be increased as needed.

In some embodiments, in the method according to the present invention, the lignocellulose substrate comprises about 50% (w/w) or more food by-product in its composition. In some embodiments, the lignocellulose substrate comprises about 70% (w/w) or more food by-product. In some embodiments, the lignocellulose substrate is comprised of food by-product. In preferred embodiments, the lignocellulose substrate is comprised of plant-based food by-product.

In some embodiments, the lignocellulose substrate itself may serve as a source of one or more nutrients, useful for growing the mycelium-producing fungi. Exemplary nutrients are carbon (for example, in the form of carbohydrate) source, nitrogen source, macronutrients and micronutrients. In some embodiments, the lignocellulose substrate is further supplemented with additional nutrients, selected from carbohydrate source, nitrogen source, macronutrients, micronutrients, enzymes, other additives and combination thereof. In some embodiments, the lignocellulose substrate is supplemented with at least one of the additional nutrients before inoculation with the mycelium-producing fungi. In some embodiments, the lignocellulose substrate may be supplemented with additional nutrients during the growth step of mycelium-producing fungi.

In some embodiments, the carbohydrate source is selected from plant origin food by-products: residues, hulls, seed cakes, leaves, stems, roots, husks, peels, spent grains, pomace from fruit and vegetables, crops (rice, wheats, maize, oats, buckwheat, sorghum and other), legumes, coffee, cacao, olives, sunflower and other oils food industries, that is the lignocellulose substrate main component food by-product serves as carbohydrate source. In other embodiments, carbohydrate source is selected from glucose and starch and combination thereof.

In some embodiments, the nitrogen source is selected from yeast extract, brewers spent grains, legumes by products and legumes starches (e.g. pea starch) and combination thereof.

In some embodiments, the macronutrients are selected from plant origin food by-products: residues, hulls, seed cakes, leaves, stems, roots, husks, peels, spent grains, pomace from fruit and vegetables, crops (rice, wheats, maize, oats, buckwheat, sorghum and other), legumes, coffee, cacao, olives, sunflower and other oils food industries.

In some embodiments, in the method according to the present invention, the lignocellulose substrate is supplemented with amylolytic and/or cellulolytic enzymes. In some embodiments, the amylolytic and/or cellulolytic enzymes are selected from $\alpha$-amylases (EC 3.2.1.1), $\beta$-amylase (EC 3.2.1.2), glucoamylase (EC 3.2.1.3), $\alpha$-glucosidase (EC 3.2.1.20), amylopullulanase (EC 3.2.1.41), cyclodextrin glycosyltransferase (EC 2.4.1.19), (endo-(1,4)-$\beta$-d-glucanase (EC 3.2.1.4), exo-(1,4)-$\beta$-d-glucanase (EC 3.2.1.91), $\beta$-glucosidases (EC 3.3.1.21), $\beta$-glucosidases (EC 3.3.1.21), and combination thereof.

In some embodiments, in the method according to the present invention, the lignocellulose substrate is supplemented with additives selected from yeast extract, magnesium ions (e.g. in salt form such as magnesium sulfate ($MgSO_4$)), calcium ions (e.g. in salt form calcium carbonate $CaCO_3$), zinc ions (e.g. zinc sulfate $ZnSO_4$) and a combination thereof.

In some embodiments, the lignocellulose substrate is supplemented with macronutrients such as primary and/or secondary metabolites and fiber. Primary metabolites may include carbohydrates, fats, non-starch polysaccharides, starches and proteins, whereas secondary metabolites are compounds, which are part of primary metabolites structure, or are catalysts or components in catabolic and metabolic pathways; for example: amino acids, di-, tri-, poly- peptides, fatty acids, mono-, di-, tri-, poly- saccharides, phenolic compounds, organic acids.

The method according to the present invention comprises a step b) inoculating the lignocellulose substrate with an effective amount of at least one mycelium-producing fungi. As a result, an inoculated lignocellulose substrate is provided. As used herein, the inoculation step generally includes any manner of adding the mycelium-producing fungi to the lignocellulose substrate so that subsequent steps can be carried out to grow the mycelium and form a matrix within and/or around the substrate. Generally speaking, mycelium-producing fungi is added to the substrate through the addition of spores into and/or on the substrate, though mycelium can also be added directly into the substrate. In some embodiments, inoculation may be carried out so that the mycelium-forming fungi is located throughout the substrate, both within the substrate and on the surfaces of the substrate. Any manner of inoculating the substrate with spores of the mycelium-producing fungi can be used. In some embodiments, the spores are in a suspension (for example in a growth medium) and the suspension is introduced into and on the substrate to deliver spores to different portions of the substrate. Preferably, the inoculum in a form of suspension makes up from 2 % (w/w) to 10 % (w/w) from total mass of the inoculated lignocellulose substrate, preferably, 4% (w/w).

In some embodiments, in the method according to the present invention, the lignocellulose substrate is inoculated with one or more mycelium-producing fungi species. In some embodiments, the lignocellulose substrate is inoculated with more than one mycelium-producing fungi species. In some examples, the lignocellulose substrate may be inoculated with one mycelium-producing fungi species. In other examples, the lignocellulose substrate may be inoculated with a mixture of two, three, four, five or six mycelium-producing fungi species In some embodiments, the mycelium-producing fungi are filamentous fungi species. In some embodiments, mycelium-producing fungi is selected from *Aspergillus* genus, *Rhizopus* genus, *Fusarium* genus, *Pleurotus* genus, *Lentinus* genus, *Hericium* genus and combinations thereof. In some embodiments, mycelium-producing fungi is selected from *Aspergillus sp., Rhizopus sp., Fusarium sp., Pleurotus sp., Lentinus sp., Hericium sp.* and combinations thereof. In some embodiments, the lignocellulose substrate is inoculated with a mixture of *Aspergillus spp.* and *Fusarium spp.* mycelium-producing fungi. In preferred embodiments, the mycelium-producing fungi is *Fusarium* genus, in particular, *Fusarium spp.*

The method according to the present invention comprises a step c) growing the mycelium-producing fungi to produce a fungal biomass. In some embodiments, the step c) is performed by solid-state fermentation. As used herein, the term "solid-state fermentation" refers a way when a culture of microorganisms (e.g. mycelium-producing fungi) are grown on a solid support selected for the purpose. In the present invention disclosure the solid support selected for growing the mycelium-producing fungi is the lignocellulose substrate as described herein. In a solid-state fermentation method, an inoculated substrate (e.g. the lignocellulose substrate inoculated with mycelium-producing fungi as described herein) is then left in a temperature-controlled room for several days. In some embodiments, in the method according to the present invention, the inoculated lignocellulose substrate is incubated at a temperature selected from 0°C to 47°C, preferably, the temperature is 47°C. In some embodiments, the temperature may be 4°C, 10°C, 15°C, 20°C, 24°C, 30°C, 35°C, 37°C, 42°C. The incubation to grow the mycelium-producing fungi (i.e. fermentation) may take about 2 to 7 days. Preferably, the fermentation is ongoing for 3 days. In some embodiments, the incubation takes place in a thermostat. Optionally, before inoculation step or before growing step, lignocellulose substrate moisture content is increased to 60-90%, preferably to 75-85%. In some embodiments, the humidity of the inoculated lignocellulose substrate during the incubation/growing period is kept in the range of 65% to 85%, preferably at 80%.

The method according to the present invention comprises a step d) harvesting the fungal biomass and the remaining lignocellulose substrate. Preferably, the fungal biomass and the remaining lignocellulose substrate is harvested when from 50% to 95% of the lignocellulose substrate is converted into mycelium. Preferably, the fungal biomass and the remaining lignocellulose substrate are harvested when about 95% of the substrate has been used by fungi and converted into mycelium.

In some embodiments, the method according to the present invention may further comprise a step of sterilizing the lignocellulose substrate, wherein the step is performed before step b). Methods of sterilizing the substrate are known in the art. For example, sterilization may be done using autoclave, which cycle parameters are in range 121-160 °C, preferably 121 °C, for in range 15-25min, preferably 15 min.

In some embodiments, the method according to the present invention may further comprise a step of aerating the substrate, wherein the step is performed before step b). Methods of aeration are known in the art, for example, mechanical aeration using a sieve.

EP 4 685 225 A1

In some embodiments, the method according to the present invention may further comprise a step of placing the substrate on a solid support, wherein the step is performed before or after step b). In some embodiment, the solid support is flat, in other embodiments that solid support is provided in other forms. In some embodiments, the solid support is configured to facilitate air circulation to the lignocellulose substrate, for example by having openings or gaps. This facilitates better growth of the mycelium-producing fungi in the presently provided method of solid-state fermentation. Preferably, the lignocellulose substrate is put on a solid support with openings, such as, for example, a grid. Preferably, the openings of the grid are of the size of less than 2.5 mm x 2.5 mm.

In some embodiments, the method according to the present invention may further comprise a step of stabilizing the harvested fungal biomass and the remaining lignocellulose substrate. This allows for a prolonged storage of the composition, before its use. For example, stabilizing may be done by drying (e.g. by lyophilizing) the harvested fungal biomass and the remaining lignocellulose substrate. For example, the harvested fungal biomass and the remaining lignocellulose substrate is up to 72 hours at a temperature of 50-70°C. Preferably, the moisture of such stabilized dried composition is not more than 15%. In some embodiments, a dried composition may be vacuum sealed and/or kept at 6°C until use.

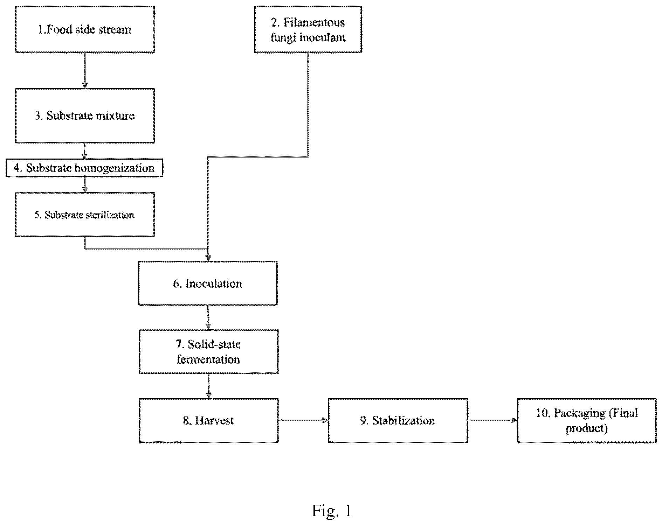

Figure 1 provides one of the embodiments of the method steps as provided by the present disclosure.

Also provided in the present invention disclosure is a composition comprising a fungal biomass, obtained by a method according to the present invention. In some embodiments, the composition comprises a fungal biomass of mycelium-producing fungi and lignocellulose substrate, wherein the composition comprises at least 30% (w/w) of the fungal biomass. In some embodiments, the composition comprises at least 12% (w/w) of protein. In some embodiments, the composition comprises from 12% (w/w) to 38% (w/w) of protein. In some embodiments, the composition comprises about 12%, about 15%, about 20%, about 25%, about 30%, about 35% or about 38% (w/w) of protein. Preferably, moisture content in said composition is about 25%. In some embodiments, the composition comprises from about 20% (w/w) to about 38% (w/w) of protein. In some embodiments, the composition comprises from about 20% (w/w) to about 38% (w/w) of protein and moisture content is about 25%.

Also provided herein is the use of the composition comprising a fungal biomass, obtained by the method according to the present invention. In particular, use of the composition in the production of food, nutraceuticals, functional food, pharmaceutical products, protein powder, nutritional supplement, higher-added nutritional value products, products claimed as source of proteins (e.g. protein bar), ready-to go meals, pet food, feed, food and feed ingredients is provided.

Also provided herein is a food product comprising the composition comprising a fungal biomass, that is obtained by the method according to the present invention. For example, harvested and lyophilized mycelium biomass may be grinded using mill, to the particles of diameter of less than 0.5 mm. The powder thus obtained may be incorporated into mushroom soup for higher protein content, also enrich pasta dough and similar products.

EXAMPLES

**Example 1. Preparation of lignocellulose substrate**

Biochemical composition of the respective food by-products was determined before use (some examples are provided in Table 1).

**Table 1**

| Sample | Carbo hydrat es, % | Energet ic value (kJ/100 g, kcal/10 0g) | Pro tein conte nt, % | Moi st ure, % | Sodiu m chlori de, % | Ashe s, % | Fat conte nt, % | Suga rs, % |
|---|---|---|---|---|---|---|---|---|
| Brewers spent grain | 13.2 | 361/86 | 4.3 | 80.1 | 0.0 | 0.7 | 1.7 | 0.5 |
| *Cannabis sativa* press cake | 16.8 | 1929/461 | 43.5 | 7.7 | 0.0 | 7.6 | 24.4 | 4.7 |
| Sugar beet press cake | 71.1 | 1353/319 | 7.3 | 5.7 | 0.0 | 15.0 | 0.3 | 2.8 |
| Carrot press cake | 79.7 | 1519/358 | 9.2 | 5.9 | 0.3 | 5.0 | 0.2 | 39.4 |
| Beet root press cake | 80.4 | 1542/363 | 10.1 | 4.3 | 0.1 | 5.1 | 0.1 | 47.0 |

To make the most suitable lignocellulose substrate, 100g of the food by-products or their mixtures were homogenized to particles of about 1.5 mm and less. For making the particles the selected by-products were dried to 8% of moisture content, milled using grain mill (Exgizmo, UPC: 619107730507) at 25.000-35.000 rpm, for 30 seconds with fractionation function of particles size <1.5 mm. In cases where moisture content was analysed, it was done using moisture analyzer PBM 53 ADAM, following instructions of use of the analyzer.

Substrates for growing the mycelium-producing fungi were prepared by mixing brewer's spent grain (BSG) and press cakes from various food by-product sources at a ratio 1:4, respectively. Moisture content was increased to 80%. For specific growth batches, 0.5% (w/w) of calcium carbonate, 0.5% of magnesium sulfate and/or 0.3% of yeast extract was added.

Before the inoculation lignocellulose substrates were sterilized using autoclave, at 121 °C for 15 min. The temperature is to inactivate spores of *Clostridium botulinum, Cl. sporogenes, Cl.perfringers, Clostridium putrificum.* Same sterilization cycle was applied for every tool, which further used for inoculation. After sterilization microbial content was checked to conform to requirements as per COMMISSION REGULATION (EC) No 2073/2005 of 15 November 2005 on microbiological criteria for foodstuffs and other regulations. Exemplary test results are provided in Table 2.

**Table 2**. Microbial evaluation requirements after sterilization for food side stream.

| Microbial evaluation | Sampling plan | | Limits | | Analytical reference method |
|---|---|---|---|---|---|
| | n | c | m | M | |
| *Clostridium botulinum, Cl. sporogenes, Cl.perfringers, Cl, putrificum* | 5 | 0 | Absence in 25g | | ISO/TS 17919:2013 |
| *E. Coli* | 5 | 0 | Absence in 25g | | ISO 16649 -1 or 2 |
| *Salmonella* | 5 | 0 | Absence in 25g | | EN/ISO 6579 |
| Aerobic colony count | 5 | 0 | <1 | | ISO 4833 |
| Mesophilic lactic acid bacteria count | 5 | 0 | <1 | | LST ISO 15214:2009N |
| Yeast and mold | 5 | 0 | <1 | | LST ISO 21527-1:2008 |
| n = number of units comprising the sample; c = number of sample units giving values over m or between m and M. | | | | | |

After sterilization, in order to provide more exposed surface for growth of mycelium-producing fungi, the substrate was aerated using a mechanical sieve.

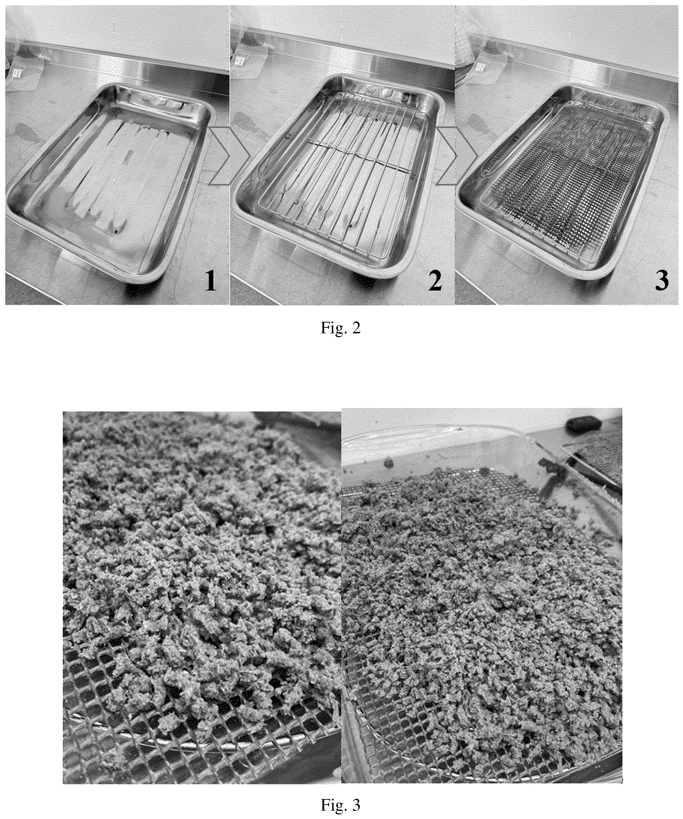

Solid state fermentation conditions were prepared as follows. Into a sheet pan made of stainless steel (Grade 316, 316 SS or 430) (Fig. 2, 1) a stainless steel (Grade 316, 316 SS or 430) grid is placed (Fig. 2, 2), and an upper food grade grid (one square size <2.00x2.00 mm, polytetrafluoroethylene (PTFE) food grade Teflon material (Fig. 2, 3) is placed on the top. The sterilized and aerated substrate is evenly placed on constructed sheet pan (Fig. 3).

**Example 2. Preparation of inoculum and inoculation**

The inoculum was prepared and substrate was inoculated in a sterilized area in a laminar flow hood. In general, all surfaces were sterilized using sprays, which inactivate environmental spores, e.g. 6% hydrogen peroxide (Contec) or an alternative.

Filamentous fungi inoculum were used for inoculation. Inoculum included *Aspergillus species (sp.), Rhizopus sp., Fusarium sp. and Pleurotus sp.* or their mixtures. The inoculum was obtained from https://mycelia.be. The inoculum was sprayed from a syringe onto the prepared lignocellulose substrate spread on a grid in a sheet pan. Inoculum comprised 4% (w/w) of total mass of inoculated lignocellulose substrate.

**Example 3. Growing of fungi biomass on substrate**

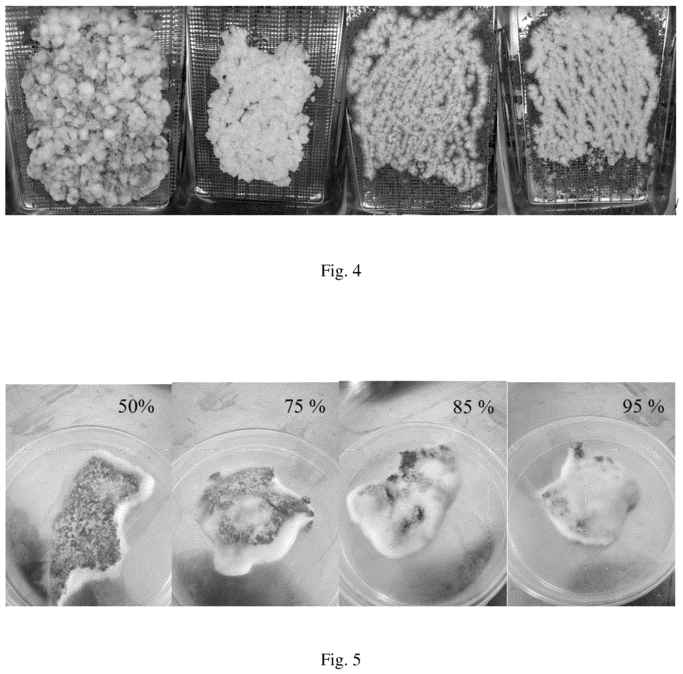

The inoculated substrate (pH 4.5, moisture 75%) was incubated to grow the fungal biomass in the thermostat with parameters: 47 °C, humidity 80%. Solid-state fermentation was ongoing for 3 days (Fig. 4). The protein biomass substrate is ready to harvest when 50-95% of substrate is converted into mycelium (Fig. 5). In most instances the fungal biomass was harvested when 95% of the substrate was used by fungi and converted into mycelium.

The harvested fungi biomass together with the remaining substrate was stabilized using extended drying up to 72 hours at 50-70 °C. Moisture content of dried stabilized biomass is up to 15%. Dried biomass was vacuum sealed and kept at 6 °C before use.

**Example 4. Analysis of biomass**

The protein content was measured before and after solid state fermentation using different food by-products and their mixtures as lignocellulose substrate (Table 3) The analysis was performed using Kjeldahl method.

Kjeldahl method:

To a Kjeldahl flask, 1.000 ±0.002 g of raw plant material (0.5 mm fraction), and 1.000±0.002 g of roasted plant material (particle size 0.5 mm) 20 ml of 98 % conc. $H_2SO_4$ and catalyst tablet (3.5 g $K_2SO_4$ and 0.4 g $CuSO_4$) were added. Content was mineralized until solution in the flask became transparent (heating intensity 60%, time - 90 min) and distilled with automatic steam distillation system (3 s NaOH and 3 s $H_3BO_4$ filing parameters, distillation time 300 min, steam intensity 80%). Distillate was collected in flask, followed with the addition of Tashiro indicator and titrated with 0.01 N HCl until the colour change from light green to grey-violet. Control sample (water) was prepared and analysed under the above described conditions. The nitrogen content, expressed as a percentage, was calculated using the following formula:

$$X=(0.014 \times A/m) \times 100 \ (g/100g)$$

A - 0.1N HCl amount, used for distillate titration, ml; m - sample weight, g; 0.0014 - nitrogen amount equivalent 1 ml 0.1 N HCl. Total nitrogen amount is calculated by multiplying the amount of nitrogen from the conversion factor 6.25.
The protein amount measurement results are provided in Table 3.
Abbreviations as used in Table 3:

B - substrate made from brewer's spent grains and beet root press cake;

M - substrate made from brewer's spent grains and carrot press cake;

CR - substrate made from brewer's spent grains and sugar beet press cake;

K - substrate made from brewer's spent grains and *Cannabis sativa* press cake;

CC - substrate made from brewer's spent grains and coffee chalks;

ZK - substrate made from brewer's spent grains and pea starch;

R - substrate made from brewer's spent grains and rapeseeds press cake;

L - substrate made from brewer's spent grains and linseeds press cake;

KM - substrate made from brewer's spent grains and hemp protein residues;

BSG-R - substrate made from brewer's spent grains (1st brewery);

CA - substrate made from brewer's spent grains (2nd brewery).

**Table 3**. Protein content before and after solid state fermentation using different food by-product mixtures as a lignocellulose substrate

| No. | Substrate composition | Additives | | | *Fungal strains* | | | Protein content before solid state fermentation, % | Protein content after solid state fermentation, % |
|---|---|---|---|---|---|---|---|---|---|
| | | Magnesium sulfate | Calcium carbonate | Yeast extract, | *Pleurotus spp.* | *Aspergillus spp.* | *Fusarium spp.* | | |
| 1. | Substrate mixture 1 (B) | + | + | + | | + | + | 9.83 | 27.22 |
| 2. | Substrate mixture 2 (M) | + | + | + | | + | + | 9.10 | 25.91 |
| 3. | Substrate mixture 3 (CR) | + | + | + | | + | + | 7.21 | 26.15 |
| 4. | Substrate mixture 4 (K) | + | + | + | | + | + | 12.11 | 37.8 |
| 5. | Substrate mixture 1 (B) | | + | + | | | + | 9.83 | 21.25 |
| 6. | Substrate mixture 2 (M) | | + | + | | | + | 9.10 | 23.11 |
| 7. | Substrate mixture 3 (CR) | | + | + | | | + | 7.21 | 23.55 |
| 8. | Substrate mixture 4 (K) | | + | + | | | + | 12.11 | 34.99 |
| 9. | Substrate mixture 1 (B) | | | + | + | | | 9.83 | 27.20 |
| 10. | Substrate mixture 2 (M) | | | + | + | | | 9.10 | 25.40 |
| 11. | Substrate mixture 3 (CR) | | | + | + | | | 7.21 | 24.03 |
| 12. | Substrate mixture 4 (K) | | | + | + | | | 12.11 | 29.89 |
| 13. | Substrate mixture 5 (CC) | | | + | | | + | 11.88 | 31.17 |
| 14. | Substrate mixture 6 (ZK) | | | + | | | + | 12.88 | 30.95 |
| 15. | Substrate mixture 7 (R) | | | + | | | + | 18.66 | 29.28 |
| 16. | Substrate mixture 8 (L) | | | + | | | + | 17.45 | 28.66 |
| 17. | Substrate mixture 9 (KM) | + | + | + | | + | + | 18.77 | 34.47 |
| 18. | Substrate mixture 10 (BSG-R) | | + | | + | | | 17.66 | 22.28 |
| 19. | Substrate mixture 11 (CA) | | + | | + | | | 11.39 | 15.58 |
| 20. | Substrate mixture 10 (BSG-R) | | + | | | | + | 17.66 | 20.77 |
| 21. | Substrate mixture 11 (CA) | | + | | | | + | 11.39 | 15.69 |

Amino acids profile

Amino acid quantitative analysis was performed at Hamilton Sekargas laboratory using high pressure liquid chromatography method PB-53/HPLC ed. II of 30.12.2008. The results are provided in Table 4.

**Table 4.** Biochemical composition and amino acids profile (grams per 100 g) of dried higher mycelium-based protein biomass.

|  | Substrate mixture 1 (B) | Substrate mixture 2 (M) | Substrate mixture 3 (CR) | Substrate mixture 4 (K) |
|---|---|---|---|---|
| Moisture % (w/w) | 5.59 | 6.38 | 6.24 | 5.41 |
| Nitrogen content % (w/w) | 5.97 | 5.45 | 4.68 | 5.83 |
| Amino acid content |  |  |  |  |
| Aspartic acid | 4.18 | 3.3 | 3.2 | 4.39 |
| Glutamic acid | 7.86 | 6.37 | 6.17 | 7.85 |
| Serine | 1.74 | 1.47 | 1.21 | 1.71 |
| Glycine | 1.41 | 1.24 | 0.96 | 1.41 |
| Histidine | 0.42 | 0.33 | 0.25 | 0.52 |
| Threonine | 1.55 | 1.44 | 1.17 | 1.54 |
| Alanine | 1.87 | 1.84 | 1.44 | 1.85 |
| Arginine | 2.16 | 1.32 | 1.12 | 2.17 |
| Proline | 2.97 | 4.09 | 2.85 | 4.15 |
| Cysteine | 2.1 | 1.69 | 1.62 | 1.64 |
| Tyrosine | 1.17 | 1.04 | 0.84 | 1.15 |
| Valine | 1.74 | 1.72 | 1.48 | 1.83 |
| Methionine | 0.78 | 0.56 | 0.38 | 0.64 |
| Lvsine | 1.65 | 1.62 | 1.3 | 1.64 |
| Isoleucine | 1.44 | 1.38 | 1.14 | 1.55 |
| Leucine | 2.77 | 2.59 | 2.13 | 2.84 |
| Phenylalanine | 2.04 | 1.88 | 1.6 | 2.1 |

## Example 5. Further embodiments

The following numbered clauses present some embodiments and combinations thereof. Further features from the specification may be combined with one or more of the items.

Clause 1. A method for the production of a composition comprising a fungal biomass, comprising the steps:

a) providing a lignocellulose substrate,
b) inoculating the lignocellulose substrate with at least one mycelium-producing fungi,
c) growing the mycelium-producing fungi to produce a fungal biomass,
d) harvesting the fungal biomass and the remaining substrate.

Clause 2. The method of clause 1, wherein the lignocellulose substrate comprises particles having a diameter of 1.5 mm or less.

Clause 3. The method of clause 1 or 2, wherein the lignocellulose substrate comprises food by-product, preferably, a plant-based food by-product.

Clause 4. The method of any of the preceding clause, wherein the food by-product is selected from plant husks, bran, pomace, oil press cake, hulls, leaves, spent grains, peels and combinations thereof.

Clause 5. The method of any of the preceding clause, wherein the food by-product is selected from brewers and other crop industries spent grain, hemp press cake, sugar beet press cake, carrot press cake, beet root press cake, coffee hulls and used grounds, legumes and potatoes starch-rich liquids, and combinations thereof.

Clause 6. The method of any of the preceding clause, wherein the lignocellulose substrate is comprised of food by-product, preferably, of a plant-based food by-product.

Clause 7. The method of any of the preceding clause, wherein the lignocellulose substrate is further supplemented with additional nutrients, selected from carbohydrate source, nitrogen source, macronutrients, micronutrients, enzymes, other additives and combinations thereof.

Clause 8. The method of clause 7, wherein the carbohydrate source is selected from glucose and starch and combination thereof.

Clause 9. The method of clause 7 or 8, wherein the nitrogen source is selected from yeast extract, brewers spent grains, legumes by-products and legumes starches and combinations thereof.

Clause 10. The method of any of clause 7 to 9, wherein the macronutrients are selected from plant origin food by-products such as residues, hulls, seed cakes, leaves, stems, roots, husks, peels, spent grains, pomace from fruit and vegetables, crops such as rice, wheats, maize, oats, buckwheat, sorghum and other, legumes, coffee, cacao, olives, sunflower and other oils food industries.

Clause 11. The method of any of the preceding clause, wherein the lignocellulose substrate is supplemented with amylolytic and/or cellulolytic enzymes.

Clause 12. The method of any of the preceding clause, wherein the amylolytic and/or cellulolytic enzymes are selected from $\alpha$-Amylases (EC 3.2.1.1), $\beta$-amylase (EC 3.2.1.2), glucoamylase (EC 3.2.1.3), $\alpha$-glucosidase (EC 3.2.1.20), amylopullulanase (EC 3.2.1.41), cyclodextrin glycosyltransferase (EC 2.4.1.19), (endo-(1,4)-$\beta$-d-glucanase (EC 3.2.1.4), exo-(1,4)-$\beta$-d-glucanase (EC 3.2.1.91), $\beta$-glucosidases (EC 3.3.1.21), $\beta$-glucosidases (EC 3.3.1.21), and combination thereof.,

Clause 13. The method of any of the preceding clause, wherein the lignocellulose substrate is supplemented with additives selected from yeast extract, magnesium ions, calcium ions, zinc ions, and combination thereof.

Clause 14. The method of any of the preceding clause, further comprising a step of sterilizing the substrate, wherein the step is performed before step b).

Clause 15. The method of any of the preceding clause, further comprising a step of aerating the substrate, wherein the step is performed before step b).

Clause 16. The method of any of the preceding clause, further comprising a step of placing the substrate on a solid support, wherein the step is performed before or after step b).

Clause 17. The method of clause 16, wherein the solid support is configured to facilitate air circulation to the substrate.

Clause 18. The method of any of the preceding clause, further comprising a step of stabilizing the harvested fungal biomass and the remaining substrate.

Clause 19. The method of any of the preceding clause, wherein the substrate is inoculated with more than one mycelium-producing fungi species.

Clause 20. The method of any of the preceding clause, wherein the mycelium-producing fungi is selected from *Aspergillus* genus, *Rhizopus* genus, *Fusarium* genus, *Pleurotus* genus, *Lentinus* genus, *Hericium* genus and combinations thereof.

Clause 21. The method of any of the preceding clause, wherein the mycelium-producing fungi is *Fusarium* genus.

Clause 22. A composition comprising a fungal biomass, obtained by a method according to any preceding clauses.

Clause 23. The composition according to clause 22, wherein the composition comprises a fungal biomass of mycelium-producing fungi and lignocellulose, wherein the composition comprises at least 30% (w/w) of the fungal biomass.

Clause 24. The composition according to clause 22 or 23, wherein the composition comprises at least 12% (w/w) of protein.

Clause 25. Use of the composition of any of clauses 22 to 24 in the production of food, nutraceuticals, functional food, pharmaceutical products, protein powder, nutritional supplement, higher-added nutritional value products, products claimed as source of proteins, ready-to go meals, pet food, feed, food and feed ingredients.

Clause 26. A food product comprising the composition according to any of clauses 22 to 24.

References

1. Xue, L., Liu, G., Parfitt, J., Liu, X., Van Herpen, E., Stenmarck, Å., O'Connor, C., Östergren, K., & Cheng, S. (2017). Missing food, missing data? A critical review of global food losses and Food Waste Data. Environmental Science & Technology, 51(12), 6618-6633. https://doi.org/10.1021/acs.est.7b00401

2. Streimikyte P, Viskelis P, Viskelis J. Enzymes-Assisted Extraction of Plants for Sustainable and Functional Applications. Int J Mol Sci. 2022 Feb 21;23(4):2359. doi: 10.3390/ijms23042359. PMID: 35216475; PMCID: PMC8876524.

3. Wikandari, R., Manikharda, Dewanti-Hariyadi, R., & Taherzadeh, M. J. (2023). Filamentous fungi for food. Current Developments in Biotechnology and Bioengineering, 343-397. https://doi.org/10.1016/b978-0-323-91872-5.00007-7

**Claims**

1. A method for the production of a composition comprising a fungal biomass, comprising the steps:

   a) providing a lignocellulose substrate,
   b) inoculating the lignocellulose substrate with at least one mycelium-producing fungi,
   c) growing the mycelium-producing fungi to produce a fungal biomass,
   d) harvesting the fungal biomass and the remaining substrate.

2. The method of claim 1, wherein the lignocellulose substrate comprises particles having a diameter of 1.5 mm or less.

3. The method of claim 1 or 2, wherein the lignocellulose substrate comprises food by-product, preferably, a plant-based food by-product.

4. The method of any of the preceding claim, wherein the food by-product is selected from plant husks, bran, pomace, oil press cake, hulls, leaves, spent grains, peels and combinations thereof, preferably wherein the food by-product is selected from brewers and other crop industries spent grain, hemp press cake, sugar beet press cake, carrot press cake, beet root press cake, coffee hulls and used grounds, legumes and potatoes starch-rich liquids, and combinations thereof.

5. The method of any of the preceding claim, wherein the lignocellulose substrate is further supplemented with additional nutrients, selected from carbohydrate source, nitrogen source, macronutrients, micronutrients, enzymes, other additives and combinations thereof.

6. The method of any of the preceding claim, wherein the lignocellulose substrate is supplemented with additives selected from yeast extract, magnesium ions, calcium ions, zinc ions, and combination thereof.

7. The method of any of the preceding claim, further comprising a step of aerating the substrate, wherein the step is performed before step b).

8. The method of any of the preceding claim, further comprising a step of placing the substrate on a solid support, wherein the step is performed before or after step b).

9. The method of claim 8, wherein the solid support is configured to facilitate air circulation to the substrate.

10. The method of any of the preceding claim, further comprising a step of stabilizing the harvested fungal biomass and the remaining substrate.

11. The method of any of the preceding claim, wherein the mycelium-producing fungi is selected from *Aspergillus* genus, *Rhizopus* genus, *Fusarium* genus, *Pleurotus* genus, *Lentinus* genus, *Hericium* genus and combinations thereof.

12. A composition comprising a fungal biomass, obtained by a method according to any preceding claims.

13. The composition according to claim 12, wherein the composition comprises a fungal biomass of mycelium-producing fungi and lignocellulose, wherein the composition comprises at least 30% (w/w) of the fungal biomass, and/or wherein the composition comprises at least 12% (w/w) of protein.

14. Use of the composition of claim 12 or 13 in the production of food, nutraceuticals, functional food, pharmaceutical products, protein powder, nutritional supplement, higher-added nutritional value products, products claimed as source of proteins, ready-to go meals, pet food, feed, food and feed ingredients.

15. A food product comprising the composition according to claim 12 or 13.

Fig. 1

Fig. 2

Fig. 3

Fig. 4

Fig. 5

Fig. 6

Europäisches
Patentamt

European
Patent Office

Office européen
des brevets

# EUROPEAN SEARCH REPORT

Application Number

EP 24 19 0312

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WINQUIST E ET AL: "Production of lignin modifying enzymes on industrial waste material by solid-state cultivation of fungi", BIOCHEMICAL ENGINEERING JOURNAL, ELSEVIER, AMSTERDAM, NL, vol. 42, no. 2, 1 November 2008 (2008-11-01), pages 128-132, XP024523818, ISSN: 1369-703X, DOI: 10.1016/J.BEJ.2008.06.006 [retrieved on 2008-06-20] * Whole doc., in partic. * | 1,2,5, 8-12 | INV. C12N1/14 A23J1/00 A23J3/20 A23K10/12 A23K10/30 A23L7/104 A23L31/00 |
| X | WO 2018/100101 A1 (UNIV COLLEGE DUBLIN NAT UNIV IRELAND DUBLIN [IE] ET AL.) 7 June 2018 (2018-06-07) * Whole doc., in partic. Materials and Methods section * | 1-3,5,6, 8-13 | |
| X | US 2016/264926 A1 (WINISKI JACOB [US] ET AL) 15 September 2016 (2016-09-15) * Whole doc., in partic. Example 1, points 1,2,3(a,b,c),5,6 * | 1-3,5,6, 8-13 | TECHNICAL FIELDS SEARCHED (IPC) C12N A23L C11C |
| X | CHMELOVÁ DANIELA ET AL: "The production of laccases by white-rot fungi under solid-state fermentation conditions", WORLD JOURNAL OF MICROBIOLOGY AND BIOTECHNOLOGY, SPRINGER NETHERLANDS, DORDRECHT, vol. 38, no. 2, 6 January 2022 (2022-01-06), XP037658789, ISSN: 0959-3993, DOI: 10.1007/S11274-021-03207-Y [retrieved on 2022-01-06] * Whole doc., in partic. p.3/4 of 20; Table 1 * | 1-6,8, 10-12 | A23J A23K |

-/--

The present search report has been drawn up for all claims

| Place of search | Date of completion of the search | Examiner |
|---|---|---|
| Munich | 13 December 2024 | Roscoe, Richard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

**Application Number**

EP 24 19 0312

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DE 10 2012 001682 A1 (CHIDO UG HAFTUNGSBESCHRAENKT [DE]) 25 July 2013 (2013-07-25) * Whole doc., in partic. para. [0045,0049] * ----- | 1-5,8-12 | |
| X | RU 2 670 526 C2 (FEDERALNOE GOSUDARSTVENNOE BYUDZHETNOE OBRAZOVATELNOE UCHREZHDENIE VYS) 23 October 2018 (2018-10-23) * Whole doc., in partic. Example 1 * ----- | 1-15 | |

TECHNICAL FIELDS SEARCHED (IPC)

The present search report has been drawn up for all claims

| Place of search | Date of completion of the search | Examiner |
|---|---|---|
| Munich | 13 December 2024 | Roscoe, Richard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.........................................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 19 0312

This annex lists the patent family members relating to the patent documents cited in the above-mentioned European search report.
The members are as contained in the European Patent Office EDP file on
The European Patent Office is in no way liable for these particulars which are merely given for the purpose of information.

13-12-2024

| Patent document cited in search report | Publication date | Patent family member(s) | Publication date |
|---|---|---|---|
| WO 2018100101 A1 | 07-06-2018 | NONE | |
| US 2016264926 A1 | 15-09-2016 | US 2016264926 A1<br>WO 2016149002 A1 | 15-09-2016<br>22-09-2016 |
| DE 102012001682 A1 | 25-07-2013 | NONE | |
| RU 2670526 C2 | 23-10-2018 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **XUE, L.** ; **LIU, G.** ; **PARFITT, J.** ; **LIU, X** ; **VAN HERPEN, E.** ; **STENMARCK, Å** ; **O'CONNOR, C.** ; **ÖSTERGREN, K** ; **CHENG, S.** Missing food, missing data? A critical review of global food losses and Food Waste Data.. *Environmental Science & Technology*, 2017, vol. 51 (12), 6618-6633, https://doi.org/10.1021/acs.est.7b00401

- **STREIMIKYTE P** ; **VISKELIS P** ; **VISKELIS J.** Enzymes-Assisted Extraction of Plants for Sustainable and Functional Applications.. *Int J Mol Sci.*, 21 February 2022, vol. 23 (4), 2359

- **WIKANDARI, R.** ; **MANIKHARDA, DEWANTI-HARIYADI, R** ; **TAHERZADEH, M. J.** Filamentous fungi for food. Current Developments. *Biotechnology and Bioengineering*, 2023, 343-397, https://doi.org/10.1016/b978-0-323-91872-5.00007-7